(19) Europäisches Patentamt
European Patent Office
Office européen des brevets

(11) Veröffentlichungsnummer: **0 249 841**
**A1**

(12) **EUROPÄISCHE PATENTANMELDUNG**

(21) Anmeldenummer: **87108231.9**

(22) Anmeldetag: **06.06.87**

(51) Int. Cl.4: **C 07 D 493/06**, C 07 D 471/06
//
(C07D493/06, 311:00, 311:00),
(C07D471/06, 221:00, 221:00)

(30) Priorität: **18.06.86 DE 3620332**

(43) Veröffentlichungstag der Anmeldung: **23.12.87**
**Patentblatt 87/52**

(84) Benannte Vertragsstaaten: **BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Heywang, Gerhard, Dr., Nittumer Weg 4,**
**D-5060 Bergisch-Gladbach 2 (DE)**

(54) **2.3.6.7-Tetrachlornaphthalin-1.4.5.8-tetracarbonsäure-diimid und ein Verfahren zur Herstellung von Tetrachlornaphthalintetracarbonsäure-Derivaten.**

(57) Die Erfindung betrifft ein Verfahren zur Herstellung von 2.3.6.7-Tetrachlornaphthalin-1.4.5.8-tetracarbonsäure-Derivaten der Formel I

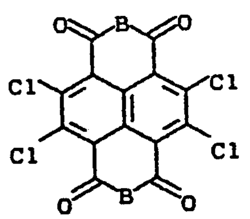

(I),

in der B für -O- oder -NH- steht, durch Oxidation von Perchlordiazapyren in saurem Medium und das neue 2.3.6.7-Tetrachlornaphthalin-1.4.5.8-tetra-carbonsäurediimid.

EP 0 249 841 A1

BAYER AKTIENGESELLSCHAFT 5090 Leverkusen, Bayerwerk
Konzernverwaltung RP
Patentabteilung B-klu/c

# 2.3.6.7-Tetrachlornaphthalin-1.4.5.8-tetracarbonsäurediimid und ein Verfahren zur Herstellung von Tetrachlornaphthalintetracarbonsäure-Derivaten

Die Erfindung betrifft ein Verfahren zur Herstellung von 2.3.6.7-Tetrachlornaphthalin-1.4.5.8-tetracarbonsäure-Derivaten der Formel I

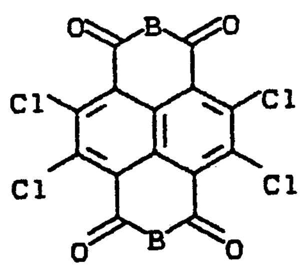

(I)

und die nach diesem Verfahren erhältliche neue Verbindung 2.3.6.7-Tetrachlornaphthalin-1.4.5.8-tetracarbonsäurediimid.

Es ist bereits bekannt, daß 2.3.6.7-Tetrachlornaphthalin-1.4.5.8-tetracarbonsäuredianhydrid durch Perchlorierung des Pyrens und anschließende oxidative Hydrolyse zugänglich ist (siehe Reimlinger und King, Chem. Ber. 95, 1046 (1962) und Vollmann, Becker, Corell, Streeck und Langbein, Liebigs Ann. Chem. 531, 1-159 (1937)). Dieses Verfahren ist, insbesondere für eine Anwendung in technischem Maßstab, außerordentlich nachteilig, weil die Herstellung des Ausgangsmaterials Dekachlorpyren über mehrere Stufen erfolgen muß und die Ausbeute bei der Hydrolyse des Perchlorpyrens nur 25 bis 30 % beträgt.

Es wurde nun gefunden, daß man 2.3.6.7-Tetrachlornaphthalin-1.4.5.8-tetracarbonsäuredianhydrid auf sehr viel einfachere Weise und in unvergleichlich besseren Ausbeuten und außerdem das bislang unbekannte entsprechende 2.3.6.7-Tetrachlor-1.4.5.8-tetracarbonsäurediimid erhalten kann, wenn man Perchlordiazapyren in saurem Medium oxydierend hydrolysiert.

Die Erfindung betrifft daher ein Verfahren zur Herstellung von 2.3.6.7-Tetrachlornaphthalin-1.4.5.8-tetracarbonsäure-Derivaten der Formel I, das dadurch gekennzeichnet ist, daß man Perchlordiazapyren in saurem Medium oxydierend hydrolysiert.

Die Erfindung betrifft ferner die neue Verbindung 2.3.6.7-Tetrachlornaphthalin-1.4.5.8-tetracarbonsäurediimid.

Das für das erfindungsgemäße Verfahren benötigte Ausgangsmaterial Perchlordiazapyren der Formel (II)

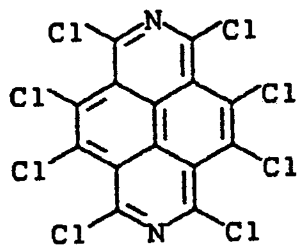

(II)

ist bekannt und auf einfache Weise, z.B. durch Perchlorierung von Naphthalin-1.4.5.8-tetracarbonsäurediimid, erhältlich (siehe DE-OS 2 148 225).

Die erfindungsgemäß oxidierende Hydrolyse des Perchlordiazapyrens der Formel (II) erfolgt durch Behandeln des Perchlordiazapyrens in einem oxidierend wirkenden sauren Medium bei Temperaturen von 20 bis 350°C, vorzugsweise 80 bis 250°C. Im allgemeinen führt man die Oxidation bei Normaldruck aus; es ist jedoch auch möglich, bei vermindertem oder erhöhtem Druck zu arbeiten, beispielsweise bei Drucken von 10 mbar bis 20 bar. Ein Arbeiten unter Druck ist beispielsweise dann angezeigt, wenn Reaktionstemperaturen angewendet werden, bei denen das oxidierend wirkende saure Medium unter Normaldruck flüchtig ist und/oder die Wirksamkeit oxidierend wirkender Gase erhöht werden soll.

Als oxidierend wirkende saure Medien können allgemein Oxidationsmittel enthaltende wasserfreie oder auch wasserhaltige Säuren verwendet werden; z.B. die Säuren der Halogene wie Fluorwasserstoff, Salzsäure, Perchlorsäure; die Säuren der Chalkogene wie Schwefelsäure,

Oleum; und die Säuren des Stickstoffs und Phosphors, wie Salpetersäure, phosphorige Säure, Ortho-phosphorsäure, Meta-phosphorsäure und Polyphosphorsäure. Falls wäßrige Säuren verwendet werden, werden diese vorzugsweise in nicht zu verdünnter Form eingesetzt. Bevorzugtes saures Medium sind Schwefelsäuren, deren Konzentration an $H_2SO_4$ über 50 Gew.-% betragen, Oleum mit $SO_3$-Gehalten von 0,1 bis 70 Gew.-% und Salpetersäuren, deren Konzentrationen an $HNO_3$ über 30 Gew.-% betragen.

Wenn das saure Medium bereits unter den Reaktionsbedingungen oxidierend wirkt, was z.B. bei Salpetersäure der Fall ist, sind keine zusätzlichen Oxidationsmittel notwendig, um ein oxidierendes saures Medium zu erhalten. Unabhängig von der verwendeten Säure kann ein oxidierendes saures Medium geschaffen werden, indem man dem sauren Medium ein beliebiges Oxidationsmittel zusetzt. Als solche Zusätze seien beispielsweise genannt: oxidierend wirkende Gase, wie Sauerstoff oder Sauerstoff enthaltende Gase, z.B. Luft, und Stickoxide und Stickoxide enthaltende Gasgemische. Ferner kommen als Oxidationsmittel in Betracht: oxidierend wirkende Metalloxide, insbesondere Übergangsmetalloxide in höheren Oxidationsstufen, wie Mangan(IV)oxid, Chrom(VI)oxid und Vanadin(V)oxid; ferner Salze, insbesondere Alkalisalze, von oxidierenden Säuren wie Permanganate, Chromate und Dichromate; Salze mit oxidierend wirkenden Kationen, wie Eisen(III)salze, Mangan(III)salze und Cer(IV)salze, insbesondere die Sulfate solcher Kationen; aktivierten Sauerstoff enthaltende Verbindungen wie Wasserstoffperoxid; und

Säuren mit oxidierenden Eigenschaften, wie Salpetersäure und Chlorsäure.

Bevorzugte Oxidationsmittel sind Sauerstoff, Luft und Salpetersäure.

Stöchiometrisch erfordert die Oxidation eines Mols Perchlordiazapyren zum 2.3.6.7-Tetrachlornaphthalintetracarbonsäure-Derivat zwei Oxidationsäquivalente. Es hat sich jedoch bewährt, das Oxidationsmittel im 2 bis 100.000-fachen Überschuß bezogen auf die stöchiometrisch erforderliche Menge einzusetzen, insbesondere bei Verwendung von gasförmigen Oxidationsmitteln.

Soll nach dem erfindungsgemäßen Verfahren 2.3.6.7-Tetrachlornaphthalin-1.4.5.8-tetracarbonsäurediimid hergestellt werden, so nimmt man die Oxidation des Perchlordiazapyrens bei tieferen Temperaturen, d.h. bei Temperaturen von 20 bis 140°C vor und verwendet starke Oxydationsmittel wie Salpetersäure, Chromsäure, Chromate und Permanganate.

Dient das erfindungsgemäße Verfahren dagegen zur Herstellung von 2.3.6.7-Tetrachlornaphthalin-1.4.5.8-tetracarbonsäuredianhydrid so führt man die Oxidation des Perchlordiazapyrens bei höheren Temperaturen, d.h. bei Temperaturen von 140 - 350°C, vorzugsweise 150 - 250°C aus. Die Oxidation des Perchlordiazapyrens zum 2.3.6.7-Tetrachlornaphthaltetracarbonsäuredianhydrid kann sowohl mit starken als auch mit schwachen Oxidationsmitteln vorgenommen werden; vorzugsweise werden

schwache Oxidationsmittel wie Luft und Sauerstoff verwendet. Das Dianhydrid kann auch durch saure Hydrolyse des Diimids erhalten werden.

Mit Hilfe des erfindungsgemäßen Verfahrens sind die 2.3.6.7-Tetrachlornaphthalin-1.4.5.8-tetracarbonsäure-Derivate der Formel I in Ausbeuten von über 90 % der Theorie, aus einem gut zugänglichen Ausgangsprodukt erhältlich.

Das erfindungsgemäße 2.3.6.7-Tetrachlornaphthalin-1.4.5.8-Tetracarbonsäurediimid bzw. die erfindungsgemäß erhältlichen 2.3.6.7-Tetrachlornaphthalin-1.4.5.8-tetracarbonsäure-Derivate der Formel (I) können als Zwischenprodukte für die Herstellung von Radikalanionensalzen der Formel (III)

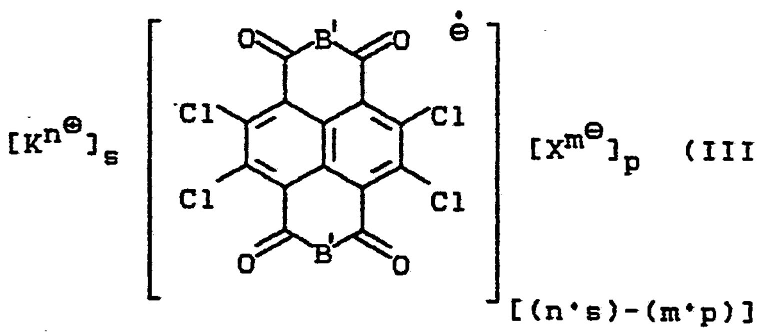

(III)

verwendet werden, in der

K für ein n-wertiges Kation steht,

n eine ganze Zahl >0 ist,

s eine ganze Zahl von 1 bis 5 ist,

X für ein m-wertiges Anion steht;

m eine ganze Zahl >0 ist,

p Null, 1 oder eine ganze Zahl >1 ist, mit der Maßgabe, daß (m·p) höchstens den Wert ((n·s)-1) annehmen darf; und

B' -O-; oder -NR- bedeutet und R für Wasserstoff oder einen gegebenenfalls substituierten Alkyl- oder Aralkyl-Rest steht.

Diese Radikalanionensalze der Formel (III) zeichnen sich durch eine sehr gute elektrische Leitfähigkeit aus und finden deshalb Verwendung zur Antistatikausrüstung von Kunststoffen und auf dem Elektroniksektor.

Die Radikalanionensalze der Formel (III) können durch elektrochemische Reduktion der 2.3.6.7-Tetrachlornaphthalintetracarbonsäure-Derivate der Formel (I) in einem inerten organischen Lösungsmittel hergestellt werden. Die elektrochemische Reduktion wird in Gegenwart eines Leitsalzes der Formel

$$[K^{n\oplus}]_s \quad [X^{m\ominus}]_p,$$

vorgenommen,

in der

K, n, s, X und m die unter Formel (III) angegebene Bedeutung haben und

p' eine ganze Zahl >1 ist, mit der Maßgabe, daß (n·s) = (m·p') ist.

Als inerte Lösungsmittel werden vorzugsweise Nitrile, Amide und Carbonate wie Dimethylformamid, Acetonitril und 1,3-Dioxa-cyclohexanon-(2) und 1,3-Dioxa-4-methylcyclopentanon-(2) verwendet.

Als Leitsalze werden vorzugsweise Methosulfate und Tetrafluoroborate der durch erschöpfende Methylierung von N,N,N',N'-Tetramethyl-1,2-diaminoethan, N,N,N',N'-Tetramethyl-1,3-diaminopropan, N,N,N',N'-Tetramethyl-1,4-diaminobutan, N,N,N',N",N"-Pentamethyl-1,7-diamino-4-azaheptan, N,N'-Dimethylpiperazin, 1,4-Diaza-[2.2.2]-bicyclooctan, Pyridin und Chinolin erhaltenen Mono- und Polykationen verwendet.

Die elektrochemische Reduktion wird bei Temperaturen von -40° C bis zum Siedepunkt des verwendeten Lösungsmittels vorgenommen. Die Konzentrationen der 2.3.6.7-Tetrachlornaphthalin-1.4.5.8-Tetracarbonsäure-Derivate der Formel (I) in den zu reduzierenden Lösungen betragen vorzugsweise 0,005 bis 0,02 Mol/l. Die Leitsalze werden vorzugsweise in einer Menge von 3 bis 12 Mol Leitsalz je Mol Bisimid der Formel (I) angewendet.

Die elektrochemische Reduktion wird vorzugsweise galvanostatisch durchgeführt. In einem typischen galvanostatischen Experiment wird in einer 100 ml-Zelle mit 2 in einem Abstand von 1 cm angeordneten 16 $cm^2$-Pt-Elektroden bei einer Stromstärke von 2 mA und einer Zellspannung zwischen 0,7 V und 3 V elektrolysiert. Gegebenenfalls können Anoden- und Kathodenraum durch eine Membran oder Fritte getrennt sein.

Die Radikalanionensalze der Formel (III) scheiden sich während der Elektrolyse an der Kathode ab und können in an sich bekannter Weise durch mechanisches Abtrennen, Waschen mit einem der oben erwähnten inerten Lösemittel und Trocknen analysenrein gewonnen werden.

Zur Herstellung der Radikalanionensalze der Formel (III), in der R für einen gegebenenfalls substituierten Alkyl- oder Aralkylrest steht werden die erfindungsgemäßen 2.3.6.7-Tetrachlornaphthalin-1.4.5.8-tetracarbonsäurediimide vor ihrer elektrochemischen Reduktion in bekannter Weise (ar)alkyliert.

## Beispiele

### Beispiel 1

1 g Perchlor-1.8-diazapyren wurde in 20 ml konzentrierter Schwefelsäure auf 200° C erhitzt und Luft durch eine Tauchfritte in das Reaktionsgemisch eingeblasen. Nach 15 Stunden ließ man auf Raumtemperatur abkühlen und goß das Reaktionsgemisch auf Eiswasser. Das ausgefallene Produkt wurde abgesaugt, mit Methanol gewaschen und bei 150° C im Vakuum getrocknet. Es wurden 0,83 g 2.3.6.7-Tetrachlornaphthalin-1.4.5.8-tetracarbonsäuredianhydrid erhalten, was einer Ausbeute von 98 % entspricht.

Die so erhaltene Verbindung verfärbte sich oberhalb von 440° C dunkel. Sie wurde durch ihr Massenspektrum eindeutig charakterisiert. Charakteristische Peaks lagen bei m/e = 404, 406, 408 und 410 im Verhältnis 7:9:4:1.

### Beispiel 2

24 g Perchlor-1.8-diazapyren wurden in 270 ml 20 %igem Oleum auf 110° C erhitzt, mit 125 ml 80 %iger Schwefelsäure verdünnt und nach dem Abkühlen auf 95 bis 100° C mit 45 ml konzentrierter Salpetersäure tropfenweise versetzt. Nach dem Abkühlen auf Raumtemperatur wurde das Reaktionsgemisch auf Eiswasser gegossen und der gebildete Niederschlag abgesaugt, mit Wasser gewaschen und im Vakuum getrocknet. Es wurden 19 g 2.3.6.7-

Tetrachlornaphthalin-1.4.5.8-tetracarbonsäurediimid erhalten, was einer Ausbeute von 93 % der Theorie entspricht.

Die Verbindung hatte einen Schmelzpunkt von 520° C, ein IR-Spektrum mit charakteristischen Banden bei 3.200, 3.100, 1.715 und 1.690 $cm^{-1}$ und ein Massenspektrum mit charakteristischen Peaks bei m/e = 402, 404, 406 und 408 im Verhältnis 7:9:4:1.

Beispiel 3

100 g Perchlor-1.8-diazapyren wurden in 1.500 ml konzentrierter Schwefelsäure bei 90° C gelöst. Das Heizbad wurde abgenommen und 187,5 ml 98 %ige Salpetersäure so zugetropft, daß die Temperatur zwischen 80 und 90° C gehalten werden konnte. Danach wurde wie im Beispiel 2 beschrieben aufgearbeitet und ein Produkt erhalten, das mit dem des Beispiels 2 identisch war. Die Ausbeute betrug 80 g entsprechend 95 % der Theorie.

Beispiel 4

15 g 2.3.6.7-Tetrachlornaphthalin-1.4.5.8-tetracarbonsäurediimid wurden in 300 ml konzentrierter Schwefelsäure 1 Stunde bei 200° C gerührt. Beim Abkühlen auf Raumtemperatur fielen 9 g 2.3.6.7-Tetrachlornaphthalin-1.4.5.8-tetracarbonsäuredianhydrid aus, das abfiltriert, gewaschen und getrocknet wurde. Durch Eintragen der Mutterlauge in Eiswasser wurden in entsprechender Weise weitere 4,8 g des Produkts erhalten. Die Ausbeute betrug somit insgesamt 13,8 g, entsprechend 91,5 % der Theorie.

Beispiel 5

In einer heizbaren 100 ml Elektrolysezelle mit zwei 16 $cm^2$-Pt-Elektroden im Abstand von 1 cm wird eine Lösung von 404 mg (1 mmol) 2.3.6.7-Tetrachlornaphthalin-1.4.5.8-tetracarbonsäurediimid und 3,32 g (9 mmol) N,N'-Dimethyl-diaza-[2.2.2]-bicyclooctan-bis-methosulfat in 100 ml Dimethylformamid bei 80°C vorgelegt. Man elektrolysiert 31 Stunden bei 1,5 mA, wobei sich eine Zellenspannung von 1.12 bis 1.2 V einstellt. An der Kathode kristallisiert das Radikalanionensalz in Form eines blauschwarzen, metallisch glänzenden Belags aus. Der Elektrodenbelag wird mit Dimethylformamid und anschließend mit Acetonitril gewaschen und getrocknet.

Ausbeute: 90 mg ( 7% der Theorie) Radikalanionensalz der Formel

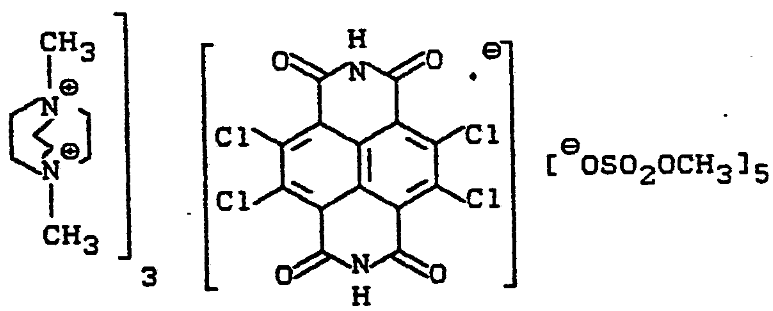

Die Leitfähigkeit des Salzes beträgt $2{,}4 \cdot 10^{-4}$ S/cm (Pulverpreßling, Zweielektrodenmethode).

Beispiel 6

In einer heizbaren 100 ml Elektrolysezelle mit zwei 16 $cm^2$-Pt-Elektroden im Abstand von 1 cm wird eine Lösung von 203 mg (0.5 mmol) 2.3.6.7-Tetrachlor-1.4.5.8-Naphthalintetracarbonsäuredianhydrid und 3,32 g (9 mmol) N,N'-Dimethyl-diaza-[2.2.2]-bicyclooctan-bis-methosulfat in 100 ml 1.2-Propylencarbonat bei 80°C vorgelegt. Man elektrolysiert 22 Stunden bei 1,5 mA, wobei sich eine Zellenspannung von 2.2 V einstellt. An der Kathode kristallisiert das Radikalanionensalz in Form kleiner schwarzer Nadeln aus. Es wird abgesaugt, mit Dimethylformamid, dann mit Acetonitril gewaschen und getrocknet.

Ausbeute: 72 mg (= 22.5% Theorie) Radikalanionensalz der Formel

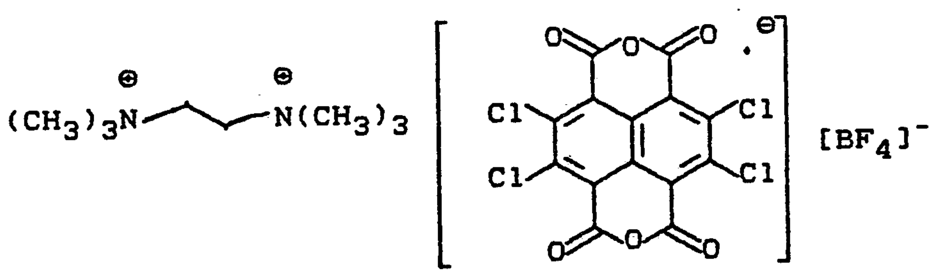

Die Leitfähigkeit des Salzes beträgt $5 \cdot 10^{-6}$ S/cm (Pulverpreßling, Zweielektrodenmethode).

Patentansprüche

1. Verfahren zur Herstellung von 2.3.6.7-Tetrachlornaphthalin-1.4.5.8-tetracarbonsäure-Derivaten der Formel

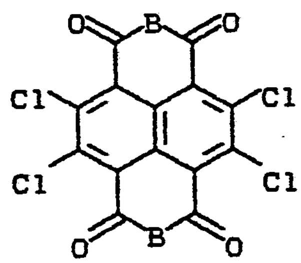

(I)

in der

B für -O- oder -NH- steht,

dadurch gekennzeichnet, daß man Perchlordiazapyren der Formel

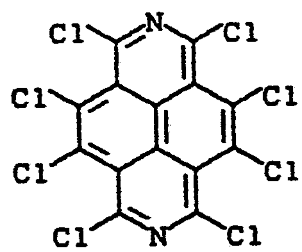

(II)

in einem sauren Medium oxydierend hydrolisiert.

2. Verfahren nach Anspruch 1, dadurch gekennzeichnet, daß man als saures Medium Säuren der Halogene, der Chalkogene, des Stickstoffs oder des Phosphors verwendet.

3. Verfahren nach Ansprüchen 1 und 2, dadurch gekennzeichnet, daß man als saures Medium Fluorwasserstoff, Salzsäure, Perchlorsäure, Schwefelsäure, Oleum, Salpetersäure, phosphorige Säure, Orthophosphorsäure, Meta-phosphorsäure oder Polyphosphorsäure verwendet.

4. Verfahren nach Ansprüchen 1 bis 3, dadurch gekennzeichnet, daß man als Oxidationsmittel oxidierende Gase, Metalloxide, Salze von oxidierenden Säuren, Salze mit oxidierend wirkenden Kationen, aktivierten Sauerstoff enthaltende Verbindungen oder Säuren mit oxidierenden Eigenschaften einsetzt.

5. Verfahren nach Ansprüchen 1 bis 4, dadurch gekennzeichnet, daß man als Oxidationsmittel Sauerstoff, Luft oder Salpetersäure verwendet.

6. Verfahren nach Ansprüchen 1 bis 5, dadurch gekennzeichnet, daß man die Oxidation bei Temperaturen von 20 bis 350°C durchführt.

7. Verfahren nach Ansprüchen 1 bis 6, dadurch gekennzeichnet, daß man es bei Drucken im Bereich von 10 mbar bis 20 bar durchführt.

8. Verfahren zur Herstellung von 2.3.6.7-Tetrachlornaphthalin-1.4.5.8-tetracarbonsäurediimid nach Ansprüchen 1 bis 7, dadurch gekennzeichnet, daß man

die Oxidation bei Temperaturen von 20 bis 140° C vornimmt und starke Oxydationsmittel verwendet.

9. Verfahren zur Herstellung von 2.3.6.7-Tetrachlornaphthalin-1.4.5.8-tetracarbonsäuredianhydrid nach Ansprüchen 1 bis 7, dadurch gekennzeichnet, daß man die Oxidation bei Temperaturen von 150 bis 300° C vornimmt.

10. 2.3.6.7-Tetrachlornaphthalin-1.4.5.8-tetracarbonsäurediimid der Formel

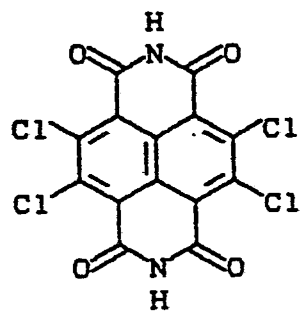

0249841

Nummer der Anmeldung

Europäisches Patentamt

## EUROPÄISCHER RECHERCHENBERICHT

EP 87 10 8231

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,A | DE-A-2 148 225 (BAYER AG)<br>* Anspruch 2; Seite 5, Formel bei Beispiel 1 *<br>--- | 1,10 | C 07 D 493/06<br>C 07 D 471/06 //<br>(C 07 D 493/06<br>C 07 D 311:00<br>C 07 D 311:00 )<br>(C 07 D 471/06<br>C 07 D 221:00<br>C 07 D 221:00 ) |
| D,A | CHEMISCHE BERICHTE, Band 95, 1962, Seiten 1043-1048, Weinheim; H. REIMLINGER et al.: "Über die Darstellung einiger Polychlor-naphthaline"<br>* Seite 1046, Zeilen 22-24 *<br>--- | 1 | |
| A | DE-A-3 000 168 (BAYER AG)<br>* Seite 15, Formel XXVI *<br>----- | 10 | |
| | | | RECHERCHIERTE SACHGEBIETE (Int. Cl.4) |
| | | | C 07 D 471/00<br>C 07 D 493/00 |

Der vorliegende Recherchenbericht wurde für alle Patentansprüche erstellt.

| Recherchenort | Abschlußdatum der Recherche | Prüfer |
|---|---|---|
| BERLIN | 14-09-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument